# EUROPÄISCHE PATENTSCHRIFT

(11) **EP 2 066 287 B1**
(45) Veröffentlichungstag und Bekanntmachung des Hinweises auf die Patenterteilung: **18.04.2012**
(21) Anmeldenummer: 07802279.5
(22) Anmeldetag: 12.09.2007
(51) Int. Cl.: A61K 8/11, A61K 8/20, A61K 8/23, A61K 8/25, A61K 8/37, A61K 8/60, A61K 8/73, A61K 8/81, A61K 8/85, A61K 8/92, A61K 8/97, A61Q 19/10

(54) **PEELINGKAPSEL MIT INTEGRIERTER PFLEGEWIRKUNG**
PEELING CAPSULE WITH INTEGRATED CARE EFFECT
CAPSULE DE PEELING AVEC EFFET SOIGNANT INTEGRE

(30) Priorität: 22.09.2006 DE 102006044942
(43) Veröffentlichungstag der Anmeldung: 10.06.2009
(73) Patentinhaber: Beiersdorf AG, 20253 Hamburg (DE)
(72) Erfinder: BEHRENS, Svea, 21109 Hamburg (DE); HOFFMANN, Nils, 22303 Hamburg (DE); WEYLAND, Silke, 67551 Worms (DE); SCHÄFER, Jessica, 22299 Hamburg (DE)
(86) Internationale Anmeldenummer: PCT/EP2007/007942
(87) Internationale Veröffentlichungsnummer: WO 2008/034549

(56) Entgegenhaltungen:
- WO-A-2005/020940
- DE-A1- 10 237 008
- JP-A- 63 185 914
- US-A1- 2004 091 446
- US-A1- 2006 127 427

## Beschreibung

Die Erfindung umfasst eine topisch applizierbare Kapsel umfassend ein Hüllmaterial auf Emulsionsbasis und ein Füllmaterial enthaltend eine abrasiv wirkende Zubereitung.

Die Epidermis setzt sich aus unterschiedlichen Zelltypen zusammen: Die Keimschicht (Stratum germinativum), die Körnerschicht (Stratum granulosum) und die Hornschicht (Stratum corneum). In der Keimschicht werden durch Teilung neue Zellen gebildet, die permanent an die Hautoberfläche wandern. Auf dem Weg dorthin, in der Körnerschicht, verhornen sie und sterben ab. Diese abgestorbenen Hornzellen machen als Hornsäulen den Großteil der Zellen in der Haut aus. Innerhalb von 28 Tagen hat sich die Epidermis einmal erneuert. Meistens lösen sich die toten Hornzellen nach und nach unmerklich und von selbst ab.

An stark beanspruchten und/oder Witterungs-/Umwelteinflüssen ausgesetzten Körperstellen, wie z.B. Hände, Füße, Ellenbogen, Gesicht oder Knie, aber auch bei sehr trockener Haut können sich vermehrt Hornschuppen ansammeln, die sich nicht von selbst lösen und so zu unschöner, matter und teils sogar ausgefranster Haut führen.

Es gibt verschiedene Möglichkeiten um die Haut von diesen überschüssigen Hornschuppen zu befreien. Zum einen gibt es verschiedene Werkzeuge, wie Bimssteine, Waschlappen oder Luffawurzeln. Zum anderen ist es weit verbreitet die Haut mit losen Körnern, wie z.B. Sand, Salz, Zucker oder Weizenschrot abzureiben. Ferner gibt es kosmetische Zubereitungen, wie Waschpeelings, die meist abrasiv wirkende Kunststoffpartikel enthalten. Um die Haut reinigen zu können, sind den meisten Waschpeelings Tenside zugesetzt. Diese Tenside entfernen jedoch nicht nur die Kunststoffpartikel, sondern auch hauteigene Lipide, die sich auf der Haut befinden und führen nach der Anwendung somit häufig zu einem spannen und Jucken der Haut.

Dem spannenden und juckenden Hautgefühl kann entgangen werden, indem der kosmetischen Peelingzubereitung Öle oder Lipide zugesetzt werden. Ein Beispiel für besonders rückfettende Peelingzubereitungen sind Öl/Salzgemische und Waschemulsionen.

Salzaufschlämmungen in Öl zur Verwendung als Peelingzubereitung sind dem Verbraucher seit einiger Zeit bekannt (Beispiel: "Großmutters Hausmittel, neu entdeckt", © 2000 Reader's Digest, Verlag Das Beste GmbH, Stuttgart, Zürich, Wien). Solche Mischungen sind ebenfalls als kosmetische Fertigprodukte im Handel erhältlich (Beispiel: Alessandro® Hands!Up Magic Manicure®). Die feinen Salzpartikel in diesen Gemischen setzen sich mit der Zeit am Boden des Behälters ab und bilden dort eine sichtbare Salzschicht. Das Vermischen der beiden Phasen kann je nach Partikelgröße des eingesetzten Salzes sehr zeit- und kraftaufwendig sein. Oftmals ist es nicht möglich eine gleichmäßige Durchmischung des Produktes zu erreichen was zur Folge hat, dass das Öl in einem schnelleren Umfang aufgebraucht wird. Zurück bleibt dann eine nahezu trockene und feste Salzschicht auf dem Boden des Behälters, welche nicht mehr verwendet werden kann.

Ansatze um diesen Missstand zu beheben werden in den Schriften WO 00/04867 und US 5,866,145 über Emulsionskapseln vorgestellt.

Wünschenswert wäre es ein Produkt bereit zu stellen, das sowohl Haut- Peeling als auch Hautpflege in einem Schritt ermöglicht.

US 20060127427 offenbart Kapseln umfassend ein Hüllmaterial und ein vom Hüllmaterial umschlossenes Füllmaterial, umfassend eine abrasiv wirkende Zubereitung, umfassend ein oder mehrere abrasiv wirkende Peelingmittel. Die Kapseln weisen eine maximale Größe von 1000 µm auf.

US 20040091446 offenbart flüssige Reinigungszubereitung. In dieser flüssigen Zubereitung können dann wiederum feste Partikel, Abrasionspartikel enthalten sein. Aufgrund von Hautbefeuchtungsmitteln in der Zubereitung kann sowohl Hautpeeling als auch Hautpflege in einem Schritt erfolgen.

WO 2005020940 offenbart dem Fachmann kapselförmige Zubereitungen für die Hautpflege. Die Kapseln umfassen eine Kapselhülle, die fest, halbfest oder formstabil ist und im Wesentlichen aus Wachsen, Emulgatoren besteht und eine Füllung aus ein oder mehreren festen, halbfesten, pastösen oder flüssigen Inhaltsstoffen umgibt.

Die Erfindung umfasst daher eine topisch applizierbare und auf der Haut verreibbare Kapsel umfassend ein Hüllmaterial auf Emulsionsbasis und ein Füllmaterial enthaltend eine abrasiv wirkende Zubereitung umfassend ein oder mehrere abrasiv wirkende Peelingmittel.
Die Peelingmittel sind entweder in
a.) einer 01- oder Lipidmischung, welche bei 25 °C eine Viskosität zwischen 3 und 50 000 m Pa*s, bevorzugt zwischen 1000 und 8000 und ganz besonders bevorzugt zwischen 2000 und 4000 mPa*s aufweist,
b.) einer tensidhaltigen Zubereitung oder
c.) einer Emulsion enthalten.

Das Hüllmaterial besteht aus einer Emulsion und umfasst ein oder mehrere Wachse, die oberhalb 25°C fest sind. Die Kapsel weist einen durchschnittlichen Durchmesser von 3 bis 40 mm auf. Das Hüllmaterial umschließt das Hüllmaterial und umfasst ein oder mehrere Wachse, die insbesondere unterhalb 40°C fest sind. So bekommt die Außenhülle ihre Festigkeit zum Gießen, Entnehmen und eine entsprechende Lagerstabilität.

Viele Begriffe wie "Kugeln", "Kapseln", "kapselförmige Zubereitung" oder "Perlen" können grundsätzlich zur Beschreibung der erfindungsgemäßen Kapseln herangezogen werden, auch wenn diesen Begriffen unter Umständen verschiedene Bedeutungen zugeordnet werden. Insbesondere die Bedeutung des Begriffes "Kapsel" ist hierin nicht auf die genau definierten Formen, Herstellungsverfahren, Inhaltsstoffe und Anwendungsmöglichkeiten der ebenfalls "Kapseln" genannten pharmazeutischen Präparate beschränkt, schließt diese aber mit ein. Generell ist erfindungsgemäß eine Kapsel ein beispielsweise ungefähr runder oder ellipsoider, von seiner Umgebung klar unterscheidbarer Gegenstand, der bei leichtem Druck und beispielsweise durch Anfassen bei der Entnahme aus einer Verpackung, seine Form nur unwesentlich ändert.
Die kapselförmigen erfindungsgemäßen Zubereitungen können beliebige Formen haben, bevorzugt sind sie jedoch sphärisch mit einem Volumen von 0,1 bis 20 ml.
Die erfindungsgemäßen Kapseln weisen eine Größe, d.h. durchschnittlichen Durchmesser, von 3, bevorzugt 5, bis 40 mm auf. Damit sind die Kapseln einzeln handhabbar und anwendbar.
Die erfindungsgemäßen kapselförmigen Zubereitungen sind als Dragees, Kapseln, Kugeln oder Hohlkugeln bei Lagerung und Entnahme formstabil.

Die Kaspelhülle, das Hüllmaterial, ist erfindungsgemäß aus einer Emulsion aufgebaut. Aufgrund des Umstandes, dass das Hüllmaterial auf Emulsionsbasis vorliegt, erfolgt beim Verreiben der erfindungsgemäßen Kapsel auf der Haut eine Verteilung wie es der Anwender von emulsionsbasierenden Cremes gewöhnt ist.

Das Füllmaterial kann ebenso als Emulsion c.) formuliert sein. Die Füllung ist dann vorteilhaft eine O/W-, W/O-, W/O/W- Emulsion, eine Mikro- oder Nanoemulsion. Das Emulsionsfüllmaterial kann aus allen in der Kosmetik bekannten Stoffen und Zubereitungen bestehen, insbesondere O/W- oder W/O/W-, W/O-Emulsionen in Form von Cremes sind vorteilhaft.

Die Emulsion der Hülle unterscheidet sich von der Emulsion der Füllung dadurch, dass die Emulsion der Füllung Tenside und/oder Wirkstoffe behinhalten kann. Weiterhin kann die Emulsion der Füllung deutlich weniger viskos sein.

Die Vorteile der Kapselhülle auf Emulsionsbasis in Kombination mit dem erfindungsgemäßen Füllmaterial sind:
➢ Kein spannen oder Jucken nach der Anwendung
➢ Versorgung der Haut mit Lipiden und Feuchtigkeit
➢ Konviniece
➢ Angenemes Hautgefühl schon bei der Anwendung
➢ Mildere Anwendung
➢ Direktes einmassieren der Wirkstoffe/ Pflegestoffe schon bei der Anwendung
➢ Hautpeeling und Hautpflege in einer Anwendung

Die Hülle lässt sich nach bekannten Verfahren herstellen.
Es können beispielsweise aus geschmolzenem Hüllmaterial Hohlkugeln gegossen werden, die durch ein Loch in der Kugelwand mit Füllmaterial befüllt werden. Danach wird das Loch durch einen Stopfen aus Hüllmaterial verschlossen.
Eine weitere Möglichkeit der Herstellung kann über das sogenannte One-Shot-Verfahren erfolgen. Dabei werden Hüll- und Füllmaterial gleichzeitig gegossen. Die Gießmaschine dosiert beide Massen aus dem geteilten Vorratsbehälter durch eine kozentrische Ringdüse in eine Blisterform. Zuerst startet die Hüllmasse über den Ringspalt und dann folgt mit kurzer Verzögerung die Füllung über die innere Düse. So kann in einem Schritt die fertige Kapsel gegossen werden. Dieses Verfahren ist das erfindungsgemäß bevorzugte Herstellungsverfahren.

Es ist auch möglich, zunächst halbe Hohlkugeln zu gießen, diese dann zu befüllen, zur Deckung zu bringen und abschließend durch thermische Behandlung die beiden Hohlkugeln miteinander zu verschmelzen. Darüber hinaus können zwei solcher Halbkugeln hergestellt werden, wobei eine oder beide ein Loch zu späteren Befüllung aufweisen, dann zu einer Hohlkugel verschweißt und abschließend durch das Füllloch befüllt werden, dass dann wie oben beschrieben verschlossen wird.

Die Peelingmittel (Peelingkörper/ Peelingpartikel) werden bevorzugt gewählt aus der Gruppe Polyethylen, Kochsalz, Meersalz, Natriumcarbonat, Natriumhydrogencarbonat, Magnesiumsulfat, Magnesiumchlorid, Zucker, Tonerde, Sand, Kunststoffpartikel, zerstoßene oder zermahlene Kerne von z.B. Walnusschalen, Aprikosen-, Pfirsich- oder Mandelkernen.

Weitere Peelingmittel sind bekannt und einsetzbar gewählt aus der Gruppe Himalayasalz, Kunststoffpartikel aus Viskose, Cellulose, Polypropylen, Polyester, Polyethylenterephthalat (PET), Polytetraflourethylen (PTFE), Aramid, Nylon, Kevlar, Polyvinylderivate, Polyurethane, Polylactid, Polyhydroxyalkanoat, Polycarbonat, Polystyrol, Celluloseester und/oder Polyethylen, sowie schwer- bzw. unlösliche Kristalle wie z.B. Calciumsulfat, Calciumcarbonat, verkapselte oder unverkapselte Kristalle wie z.B. Calciumchlorid, Kaliumchlorid, Magnesiumchlorid, Zucker, Silikate wie z.B. Seesand, Tonerde, Schlämme, geschrotete oder gemahlene Naturprodukte wie z.B. Weizen, Leinsamen, Reis, Mais, Mandeln, Nüsse, Nussschalen, Kürbiskerne, Kümmel, geschrotete oder gemahlene natürliche Schwämme wie z.B. Luffagurke, natürliche und synthetische Wachse wie z.B. Reiskleiewachs, Carnaubawachs, Jojobawachs, Bienenwachs.

Peeling (engl. to peel, "schälen, pellen") oder Schälkur ist eine kosmetische Behandlung, bei der oberflächliche Schichten der Haut flächig entfernt werden.
Beim oberflächlichen Peeling wird die oberste Hornschicht der Haut mechanisch oder chemisch entfernt. Diese Behandlung wird umgangssprachlich als Peeling bezeichnet, viele Anbieter bezeichnen diese Methode als Mikrodermabrasion.

Ein oder mehrere dieser Peelingmittel sind nun in entweder in
a.) Öl oder einer Lipidmischung, welches oder welche bei 25 °C eine Viskosität zwischen 3 und 50 000 m Pas, bevorzugt zwischen 1000 und 8000 und ganz besonders bevorzugt zwischen 2000 und 4000 mPas aufweist, oder
b.) einer tensidhaltigen Zubereitung oder
c.) einer Emulsion
enthalten und von der Kapselhülle umgeben.

Die Öl- oder Lipidphase a.), in denen die Peelingmittel suspendiert oder verteilt vorliegen können, kann gewählt werden aus der Gruppe der Ester aus gesättigten und/oder ungesättigten, verzweigten und/oder unverzweigten Alkancarbonsäuren mit einer Kettenlänge von 3 bis 30 C-Atomen und gesättigten und/oder ungesättigten, verzweigten und/oder unverzweigten Alkoholen mit einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen mit einer Kettenlänge von 3 bis 30 C-Atomen. Solche Esteröle können dann vorteilhaft gewählt werden aus der Gruppe Octylpalmitat, Ethylhexyl Cocoate Octylisostearat, Octyldodeceylmyristat, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearät, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleyleru-cat, Erucyloleat, Erucylerucat, Tridecylstearat, Tridecyltrimellitat sowie synthetische, halbsynthetische und natürliche Gemische solcher Ester. Ferner kann die Ölphase vorteilhaft gewählt werden aus der Gruppe der Dialkylether und Dialkylcarbonate. Die Ölphase kann ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan, C14-C20 Isoparaffine. Unter den Polyolefinen sind Polydecene und hydrierte Polyisobutene die bevorzugten Substanzen.

Die tensidhaltige Zubereitung b.) umfasst, neben den Peelingmittel, Tenside oder waschaktive Substanzen.
Tenside sind amphiphile Stoffe, die organische, unpolare Substanzen in Wasser lösen können. Sie sorgen, bedingt durch ihren spezifischen Molekülaufbau mit mindestens einem hydrophilen und einem hydrophoben Molekülteil, für eine Herabsetzung der Oberflächenspannung des Wassers, die Benetzung der Haut, die Erleichterung der Schmutzentfernung und -lösung, ein leichtes Abspülen und - je nach Wunsch - für Schaumregulierung.
Vorteilhafte tensidhaltige Mischungen umfassen beispielsweise
a.) 7,32 Gew.% SODIUM MYRETH SULFATE und 4,47 Gew.% Lauryl Glucoside sowie Wasser
   oder
b.) 2,82 Gew.% SODIUM MYRETH SULFATE, 11,76 Gew.% Cocamidopropyl Betaine, 4 Gew.% Decyl Glucoside und Wasser
   oder
c.) 1,8 Gew.% Sodium Methyl Cocoyl Taurate, 6,05 Gew.% Sodium Laureth Sulfate, 1 Gew.% Decyl Glucoside und Wasser.

Erfindungsgemäß besteht zum Unterschied zum Stand der Technik das Hüllmaterial nicht aus Gelantine oder reinem Wachs sondern ist emulsionsbasierend aufgebaut.

Dieser Vorteil, dass das Peelingmittel von einer hautpflegenden Emulsion umgeben ist, führt zu
- pflegende Wirkung nach dem Abspülen des Produktes und Abtrocknen des Gesichts
- kein Eincremen mehr nach der Anwendung notwendig
- Gesicht wird mit rückfettenden und pflegenden Lipiden versorgt, die vor äußeren Einflüssen wie Wind, Kälte, Heizungsluft schützten
- es entsteht kein Juckreiz oder Spannen der Haut, da die Emulsion "beruhigend" wirkt
- der zeitliche Aufwand und die Produktvielfalt verringern sich durch das "two in one" Produkt
- die Peelingkörper werden durch die Emulsion besser gedämpft, d.h. weniger Hautirritationen

Erfindungsgemäß wird eine portionierbare Darreichung eines Gesichtspeelings aufgrund der Kapselform angeboten. Die pflegenden und reinigenden Produkteigenschaften werden kombiniert in einem Produkt zur Verfügung gestellt.

Die erfindungsgemäße Kapsel ist damit zum gleichzeitigen Hautpeeling und zur Hautpflege zu verwenden.

### Beispiele

Die Zahlenangaben beziehen sich auf Gewichtsprozent jeweils bezogen auf die Gesamtmasse der Hülle bzw. der Füllung.

### A. Hüllmaterial, Emulsionshüllen

| Beispiel | 1 | 2 | 3 | 4 | 5 |
|---|---|---|---|---|---|
| Cetyl Palmitate | 4,0000 | 4,0000 | 4,0000 | 3,2000 | 3,2000 |
| Glycerin | 5,0000 | 5,0000 | 5,0000 | 5,0000 | 5,0000 |
| Hexamidine Diisethionate | 0,0800 | 0,0800 | 0,0800 | - | - |
| Hydrogenated Polydecene | 28,9267 | 28,9267 | 28,9267 | 23,1100 | 23,1100 |
| Octyldodecanol | 29,5933 | 29,5933 | 29,5933 | 23,4800 | 23,4800 |
| PEG-45/Dodecyl Glycol Copolymer | 1,6000 | 1,6000 | 1,6000 | 1,2800 | 1,2800 |
| Polyglyceryl-3 Diisostearate | 1,6000 | 1,6000 | 1,6000 | 1,2800 | 1,2800 |
| Synthetic Wax | 8,2000 | 8,2000 | 8,2000 | 6,5500 | 6,5500 |
| Tocopheryl Acetate | 1,0000 | 1,0000 | 1,0000 | 0,8000 | 0,8000 |
| Water | 20,0000 | 20,0000 | 20,0000 | 35,0000 | 35,0000 |

### B. Beispiele 6 - 10 Füllmaterial:

### 6. Waschemulsionen mit Polyethylen

| | |
|---|---|
| Acrylates/C10-30 Alkyl Acrylate Crosspolymer | 0,6000 |
| BHT | 0,0500 |
| Fragrance | 1,0000 |
| Glycine Soja (Soybean) Oil + Ricinus Communis (Castor) Seed Oil + Propyl Gallate | 9.0500 |
| Hydrolyzed Silk | 0,1000 |
| Lauryl Glucoside | 4,4700 |
| Methylparaben | 0,3500 |
| Mineral Oil | 35,0000 |
| Oenothera Biennis (Evening Primrose) Oil | 0,1000 |
| Phenoxyethanol | 0,8000 |
| Propylparaben | 0,3500 |
| Sodium Myreth Sulfate | 7,3200 |
| Polyethylen | 2,300 |
| Water + Sodium Hydroxide | 0,2500 |
| Water | q.s. |

### 7. Waschemulsionen mit Walnusschalen:

| | |
|---|---|
| Bis-Diglyceryl Polyacyladipate-2 | 1,5000 |
| Carbomer | 0,5000 |
| Ceteareth-20 | 2,0000 |
| Cetyl Alcohol | 2,2000 |
| Cetyl Palmitate | 1,0000 |
| Decyl Glucoside | 0,2500 |
| Decyl Oleate | 1,0000 |
| Fragrance | 0,3000 |
| Glycerin | 4,3500 |
| Glyceryl Stearate | 1,5000 |
| Glycine Soja (Soybean) Oil + Calendula Officinalis Flower Oil | 0,2000 |
| Isopropyl Palmitate | 7,5000 |
| Lactose + Cellulose + Ultramarines + Hydroxypropyl Methylcellulose + Tocopheryl Acetate | 0,2500 |
| Methylparaben | 0,2000 |
| Methylpropanediol | 2,0000 |
| Mineral Oil | 4,5000 |
| PEG-20 Glyceryl Stearate | 2,5000 |
| Phenoxyethanol | 0,6000 |
| Finely grounded walnut shell (peeling) | 4,3000 |
| Prunus Amygdalus Dulcis (Sweet Almond) Oil | 0,2000 |
| Water | 62,8500 |
| Water + Sodium Hydroxide | 0,3000 |

### 8.

| | |
|---|---|
| Acrylates/C10-30 Alkyl Acrylate Crosspolymer | 0,6000 |
| BHT | 0,0500 |
| Fragrance | 1,0000 |
| Glycine Soja (Soybean) Oil + Ricinus Communis (Castor) Seed Oil + Propyl Gallate | 9,0500 |
| Hydrolyzed Silk | 0.1000 |
| Lauryl Glucoside | 4,4700 |
| Methylparaben | 0,3500 |
| Mineral Oil | 35,0000 |
| Oenothera Biennis (Evening Primrose) Oil | 0,1000 |
| Phenoxyethanol | 0,8000 |
| Polyethylene | 4,3000 |
| Propylparaben | 0,3500 |
| Sodium Myreth Sulfate | 7,3200 |
| Water | 36,2600 |
| Water + Sodium Hydroxide | 0,2500 |

### 9. Waschgel

| | |
|---|---|
| Acrylates/C10-30 Alkyl Acrylate Crosspolymer | 0,7500 |
| Benzophenone-4 | 0,0500 |
| Cocamidopropyl Betaine | 11,7600 |
| Decyl Glucoside | 4,0000 |
| Fragrance | 0,2600 |
| Hydroxypropyl Starch Phosphate + Water | 1,0000 |
| Mannitol + Microcrystalline Cellulose + Chromium Hydroxide Green + Lactic Acid | 0,3000 |
| Methylparaben | 0,3500 |
| PEG-40 Hydrogenated Castor Oil | 0,5000 |
| PEG-90 Glyceryl Isostearate + Laureth-2 | 0,1500 |
| Phenoxyethanol | 0, 9500 |
| Polyethylene | 2,0000 |
| Polyquaternium-10 | 0,1000 |
| Propylparaben | 0,3500 |
| Sodium Myreth Sulfate | 2,8200 |
| Water | 73,9600 |
| Water + Sodium Hydroxide | 0,7000 |

### 10. Öl-Salz-Füllung

| | |
|---|---|
| BHT | 0, 0500 |
| Beeswax | 0,2500 |
| C18-36 Acid Triglyceride | 5,0000 |
| Caprylic/Capric Triglyceride | 14, 5000 |
| Dicaprylyl Carbonate | 14, 5000 |
| Fragrance | 0,2000 |
| Octyldodecanol | 14, 5000 |
| Prunus Amygdalus Dulcis (Sweet Almond) Oil | 0,2500 |
| Sodium Chloride | 50,0000 |
| Stearyl Alcohol | 0,2500 |
| Theobroma Cacao (Cocoa) Seed Butter | 0,5000 |

## Patentansprüche

1. Topisch applizier- und verreibbare Kapsel umfassend ein Hüllmaterial und ein vom Hüllmaterial umschlossenes Füllmaterial, umfassend eine abrasiv wirkende Zubereitung, umfassend ein oder mehrere abrasiv wirkende Peelingmittel, wobei die Peelingmittel in
a.) einer Öl- oder Lipidmischung, welche bei 25 °C eine Viskosität zwischen 3 und 50 000 m Pa*s, bevorzugt zwischen 1000 und 8000 und ganz besonders bevorzugt zwischen 2000 und 4000 mPa*s aufweist,
b.) einer tensidhaltigen Zubereitung oder
c.) einer Emulsion enthalten sind,
**dadurch gekennzeichnet, dass** das Hüllmaterial aus einer Emulsion besteht umfassend ein oder mehrere Wachse, die oberhalb 25°C fest sind und die Kapsel einen durchschnittlichen Durchmesser von 3 bis 40 mm aufweist.

2. Kapsel nach Anspruch 1, **dadurch gekennzeichnet, dass** die Peelingmittel gewählt werden aus der Gruppe Polyethylen, Kochsalz, Meersalz. Natriumcarbonat, Natriumhydrogencarbonat, Magnesiumsulfat, Magnesiumchlorid, Zucker, Tonerde, Sand, Kunststoffpartikel, zerstoßene oder zermahlene Kerne von Walnusschalen, Aprikosen-, Pfirsich- und/oder Mandelkernen.

3. Verwendung einer Kapsel nach einem der vorstehenden Ansprüche zur gleichzeitigen Hautpeeling und -pflege.

## Claims

1. Topically applicable and grindable capsule comprising a coating material and a filling material surrounded by the coating material, comprising an abrasive preparation, comprising one or more abrasive peeling agents, where the peeling agents are present in
a.) an oil or lipid mixture which, at 25°C, has a viscosity between 3 and 50 000 mPa*s, preferably between 1000 and 8000 and very particularly preferably between 2000 and 4000 mPa*s,
b.) a surfactant-containing preparation or
c.) an emulsion,
**characterized in that** the coating material consists of an emulsion comprising one or more waxes which are solid above 25 °C and the capsule has an average diameter of from 3 to 40 mm.

2. Capsule according to Claim 1, **characterized in that** the peeling agents are selected from the group polyethylene, sodium chloride, sea salt, sodium carbonate, sodium hydrogen carbonate, magnesium sulphate, magnesium chloride, sugar, clay earth, sand, plastic particles, pounded or ground cores of walnut shells, apricot, peach and/or almond kernels.

3. Use of a capsule according to one of the preceding claims for the simultaneous peeling and care of the skin.

## Revendications

1. Capsule pouvant être appliquée localement et étendue en massant, comprenant une matière d'enveloppe et une matière de remplissage entourée par la matière d'enveloppe, comprenant une préparation à action abrasive, comprenant un ou plusieurs exfoliants à action abrasive, les exfoliants étant contenus dans
a.) un mélange huileux ou lipidique, qui présente à 25 °C une viscosité comprise entre 3 et 50 000 mPa.s, de préférence entre 1 000 et 8 000 et de façon tout particulièrement préférée entre 2 000 et 4 000 mPa.s,
b.) une préparation contenant un tensioactif ou
c.) une émulsion,
**caractérisée en ce que** la matière d'enveloppe consiste en une émulsion comprenant une ou plusieurs cires qui sont solides au-dessus de 25 °C et la capsule présente un diamètre moyen de 3 à 40 mm.

2. Capsule selon la revendication 1, **caractérisée en ce que** les exfoliants sont choisis dans le groupe constitué par le polyéthylène, le chlorure de sodium, le sel 1 de mer, le carbonate de sodium, l'hydrogénocarbonate de sodium, le sulfate de magnésium, le chlorure de magnésium, le sucre, l'alumine, le sable, des particules de matière plastique, des grains concassés ou broyés de coquilles de noix, de noyaux d'abricot, de pêche et/ou de coques d'amandes.

3. Utilisation d'une capsule selon l'une quelconque des revendications précédentes pour le peeling et le soin simultanés de la peau.
